# FASCICULE DE BREVET EUROPEEN

(11) **EP 2 061 411 B1**
(45) Date de publication et mention de la délivrance du brevet: **02.03.2011**
(21) Numéro de dépôt: 07823785.6
(22) Date de dépôt: 07.09.2007
(51) Int. Cl.: A61F 9/007

(54) **VITREOTOME PNEUMATIQUE**
PNEUMATISCHES VITREOTOM
PNEUMATIC VITREOTOME

(30) Priorité: 08.09.2006 FR 0653635
(43) Date de publication de la demande: 27.05.2009
(73) Titulaire: Corneal Innovation, 75002 Paris (FR)
(72) Inventeur: GAGNEPAIN, Cédric, F-74370 Pringy (FR); ANDRE, Jean-Marc, F-73230 Saint Alban Leysse (FR)
(74) Mandataire: Schwarz, Albin
(86) Numéro de dépôt international: PCT/FR2007/051889
(87) Numéro de publication internationale: WO 2008/029066

(56) Documents cités:
- WO-A-01/15640
- US-A1- 4 577 629
- US-A1- 4 696 298
- US-B1- 6 575 990

## Description

La présente invention a pour objet un vitréotome et plus particulièrement un vitréotome commandé de façon pneumatique.

Un vitréotome est un outil chirurgical qui permet d'intervenir sur l'humeur vitrée ou corps vitré de l'oeil d'un patient.

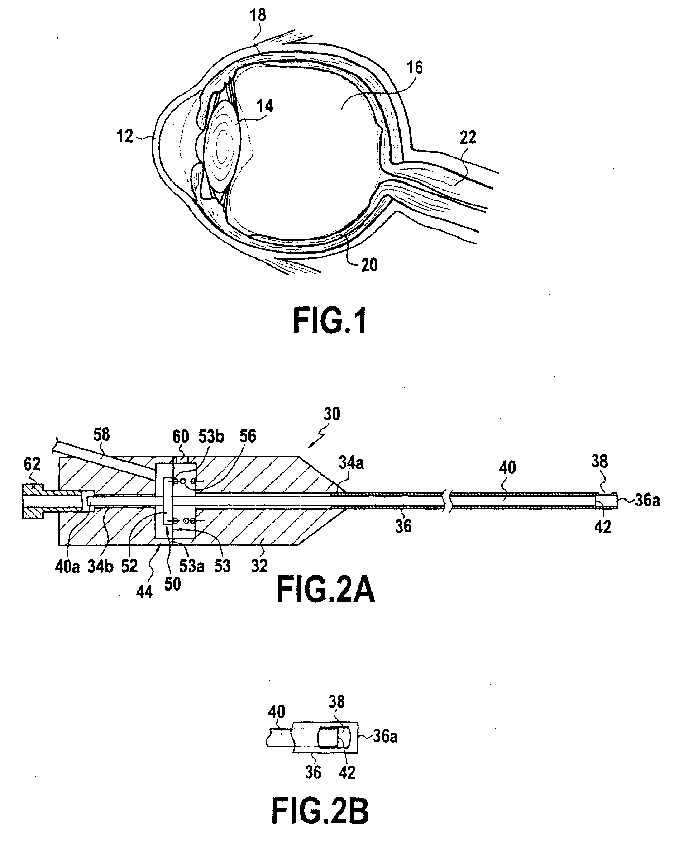

Sur la figure 1 annexée, on a représenté en coupe longitudinale un oeil. Sur cette figure, on voit plus particulièrement la cornée 12, le cristallin 14, le corps vitreux 16 disposé dans le globe oculaire 18 derrière le cristallin 14 ainsi que la rétine 20 reliée au nerf optique 22. Le corps vitré 16 est une substance transparente gélatineuse (assimilable à du blanc d'oeuf), qui remplit la cavité oculaire en arrière du cristallin. Le corps vitré est formé de 95 % d'eau et sa fonction est de donner à l'oeil sa forme et sa consistance. Il représente ainsi 98 % du volume de l'oeil. Son rôle est de garantir la rigidité du globe oculaire 18 et en particulier de maintenir la rétine 20 en place contre la paroi de l'oeil.

D'une manière simplifiée, le vitréotome comporte une canule qui peut être introduite dans l'oeil et qui est équipée d'un système d'aspiration et d'un élément mobile monté dans la canule pour découper cycliquement des petites quantités de corps vitreux aspirés dans la canule.

Le brevet américain US 5,176,628 décrit un vitréotome commandé de façon pneumatique. Dans ce vitréotome, l'organe coupant est monté rotatif à l'intérieur de la canule. La rotation alternée de l'élément coupant est obtenue grâce à la commande du déplacement alterné d'un piston par des moyens pneumatiques. Ce piston comporte une crémaillère qui coopère elle-même avec un pignon solidaire de l'outil coupant rotatif.

Un tel vitréotome comporte comme inconvénient essentiel de présenter un ensemble de commande du déplacement de l'outil coupant qui est relativement complexe et qui comporte une partie mécanique rendant la précision du déplacement de l'outil coupant relativement aléatoire et introduisant une inertie importante dans la commande du mouvement de l'outil de coupe du vitréotome.

Le document WO-A-01/15640 décrit un dispositif de vitrectomie tel que défini dans le preambule de la revendication 1.

Dans le cas d'une vitrectomie postérieure, c'est-à-dire, dans le cas d'une intervention proche de la rétine, le vitréotome doit assurer une haute vitesse de coupe avec un temps de fermeture complète de la canule le plus court et le plus stable possible même si la vitesse de coupe est très élevée. Ceci a pour but d'éviter la création d'une dépression trop importante à l'intérieur de la canule qui risquerai de blesser la rétine lors de l'ouverture suivante de la canule en raison de la dépression importante existant dans celle-ci.

Un objet de la présente invention est de fournir un vitréotome à commande pneumatique qui permet un contrôle très précis des temps d'ouverture et de fermeture de l'extrémité de la canule.

Pour atteindre ce but, selon l'invention, le dispositif de vitrectomie comprend les caractéristiques définies dans la revendication 1.

Par « directement liée », on veut dire que la liaison entre le piston du vérin et l'aiguille est réalisée de façon rigide sans qu'une pièce intermédiaire en mouvement relatif par rapport au piston ou à l'aiguille ne soit interposée.

On comprend que grâce au fait que l'aiguille dont l'extrémité constitue la partie de coupe est directement liée au piston du vérin et que ce vérin est à double effet, il est possible de commander avec précision, les mouvements de translation de l'aiguille à l'intérieur de la canule. Il est ainsi possible de contrôler avec précision le temps d'ouverture et de fermeture de la fenêtre servant à l'aspiration du corps vitré.

De préférence, ledit corps forme les deux chambres dudit vérin disposées de part et d'autre dudit piston ; et ledit piston comprend une plaque solidaire de l'extrémité proximale de ladite aiguille et un diaphragme déformable de forme annulaire dont un bord est solidaire de la périphérie de ladite plaque et dont l'autre bord est solidaire dudit corps.

De préférence également, le dispositif comprend en outre une première conduite d'alimentation de la première chambre du vérin et une deuxième conduite d'alimentation de la deuxième chambre du vérin.

De préférence encore, le dispositif comporte des moyens pour relier alternativement chaque conduite à une source de gaz sous pression et à l'échappement et des moyens de commande des moyens de mise en liaison.

De préférence également, lesdits moyens de commande commandent les moyens de liaison de telle manière que le cycle complet de déplacement de l'aiguille par rapport à la canule ait une durée constante et que, entre deux cycles consécutifs, l'extrémité distale de l'aiguille soit maintenue dans sa deuxième position.

Selon le mode de réalisation préféré défini précédemment, l'aiguille est maintenue entre deux cycles de découpe dans la position telle que la fenêtre de la canule reste ouverte, ce qui évite la création d'une dépression importante à l'intérieur de la canule. Par ailleurs, la durée du cycle complet de découpe a une durée fixe et la cadence de découpe est déterminée par les intervalles de temps séparant deux cycles de découpe.

D'autres caractéristiques et avantages de l'invention apparaîtront mieux à la lecture de la description qui suit d'un mode préféré de réalisation de l'invention donné à titre d'exemple non limitatif. La description se réfère aux figures annexées sur lesquelles :
- la figure 1 déjà décrite est une vue en coupe longitudinale d'un oeil humain ;
- la figure 2A est une vue en coupe longitudinale d'un vitréotome selon l'invention;
- la figure 2B est une vue partielle de dessus de l'extrémité de la canule du vitréotome ;
- la figure 3 est un diagramme illustrant l'alimentation en air comprimé du vérin de commande des déplacements de l'aiguille ; et
- la figure 4 montre deux cycles possibles de commande des mouvements de l'aiguille à l'intérieur de la canule.

En se référant tout d'abord aux figures 2A et 2B, on va décrire l'architecture du vitréotome.

Sur ces figures, on a représenté en coupe longitudinale le vitréotome 30. Celui-ci comprend un corps de préférence cylindrique 32 dans lequel est ménagé un passage axial 34. A l'extrémité distale 34a de ce passage est montée une canule cylindrique 36. L'extrémité distale 36a de la canule est obturée et une fenêtre 38 est ménagée dans la paroi latérale de la canule à proximité de l'extrémité distale 36a. A l'intérieur du passage axial 34 et de la canule 36 est montée mobile en translation linéaire une aiguille creuse 40 dont l'extrémité distale 42 est coupante. On comprend dès à présent que par des mouvements de translation à l'intérieur de la canule 36, l'aiguille 40 peut dégager la fenêtre 42 ou au contraire obturer celle-ci.

Les déplacements de l'aiguille 40 sont commandés par un vérin à double effet 44. Ce vérin 44 est constitué essentiellement par deux chambres 46 et 48 ménagées dans le corps 32 du vitréotome. Les chambres 46 et 48 sont séparées par un piston 50. De préférence, le piston 50 est constitué par une plaque circulaire 52 solidaire de l'aiguille 40 de préférence à proximité de son extrémité proximale 40a et par une membrane en forme de couronne 53. La plaque 52 est sensiblement perpendiculaire à l'axe de l'aiguille. Le bord externe 53a de la membrane est solidaire du corps alors que son bord interne 53b est solidaire de la périphérie de la plaque circulaire 52. En outre, un ressort de rappel 56 est interposé entre la plaque 52 et la paroi de la chambre 48. Le ressort de rappel tend à maintenir l'aiguille 40 dans sa position retirée représentée sur la figure 2A, position dans laquelle la fenêtre 38 est ouverte.

Deux conduites 58 et 60 permettent d'alimenter en gaz sous pression respectivement les chambres 46 et 48 du vérin à double effet.

L'extrémité 34b proximale du passage axial 36 est prolongée par un embout de raccordement 62, embout qui est destiné à être raccordé à une canalisation d'aspiration. Ainsi, l'effet d'aspiration est transmis à la fenêtre 38 lorsque l'aiguille occupe sa position rétractée provoquant ainsi l'introduction d'un petit volume de corps vitré dans la canule. Lorsque l'aiguille se déplace vers l'extrémité distale de la canule, son extrémité coupante sectionne le volume de vitré qui est aspiré par la dépression.

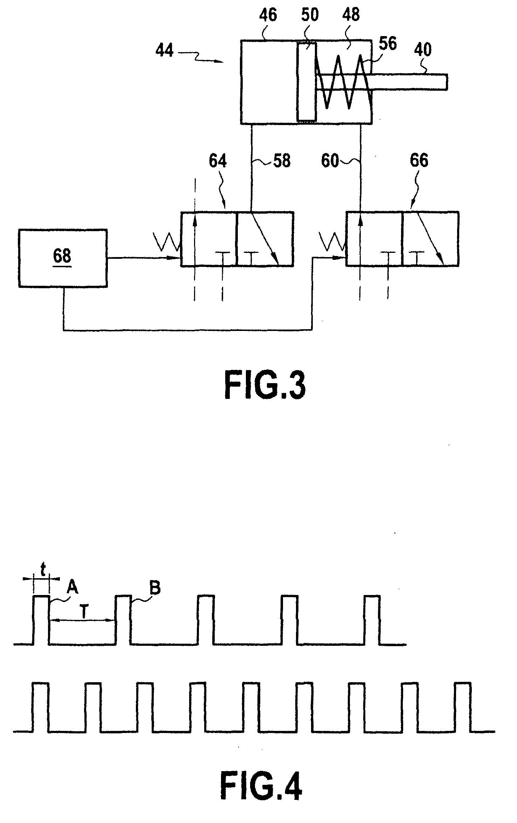

Sur la figure 3, on a représenté de façon simplifiée la commande du vérin à double effet 44. Chacune des conduites d'alimentation 58 et 60 est raccordée à une électrovanne 64 et 66 pouvant soit mettre la chambre correspondante à l'échappement, soit la raccorder à une source de gaz sous pression. La commande des électrovannes 64 et 66 est réalisée par un circuit de commande 68.

Sur la figure 4, on a représenté deux cycles possibles de fonctionnement du vitréotome. Les impulsions représentées sur les deux courbes A, B, etc., correspondent à un cycle complet d'ouverture et de fermeture de la fenêtre 38. Durant ce cycle, l'aiguille quitte sa position retirée pour venir couper le morceau de corps vitré aspiré puis fermer la fenêtre 38 et enfin revenir à sa positon initiale. Comme on le voit, la durée de ce cycle est fixe et égale à t. Par exemple t vaut 22 ms. En revanche, il est possible de régler la durée de la période T séparant deux cycles consécutifs de fonctionnement de l'aiguille. Durant les intervalles de temps T, l'aiguille est maintenue dans sa position retirée, notamment par le ressort de rappel 56.

La possibilité d'avoir un temps de fermeture très bref de la fenêtre par l'aiguille permet d'avoir une aspiration optimale.

## Revendications

1. Dispositif de vitrectomie comprenant
- un corps (32) ;
- une canule (36) s'étendant à une extrémité du corps, ladite canule présentant une extrémité distale (36a) obturée et munie d'une fenêtre (38) dans sa paroi latérale à proximité de ladite extrémité distale ;
- une aiguille (40) animée d'un mouvement alternatif dans ladite canule pour obturer périodiquement ladite fenêtre ;
- des moyens d'aspiration raccordés à ladite aiguille ; et
- un vérin (44) muni d'un piston (50) mobile pour commander le mouvement de ladite aiguille ;
ladite aiguille étant animée d'un mouvement alternatif de translation à l'intérieur de ladite canule ;
- un ressort de rappel (56) interposé entre ledit corps (32) et ledit piston (50), tendant à ramener ladite aiguille (40) dans sa deuxième position le dispositif **se caractérisant en ce que** :
- ledit vérin est à double effet ;
- le piston dudit vérin est directement solidaire de ladite aiguille et s'étend sensiblement perpendiculairement à l'axe de ladite aiguille, lesdits déplacements du piston étant aptes à amener ladite aiguille dans une première position dans laquelle ladite aiguille obture ladite fenêtre (38) et une deuxième position dans laquelle l'extrémité distale (42) de l'aiguille est écartée de l'extrémité distale (36a) de la canule pour laisser ouverte ladite fenêtre.

2. Dispositif selon la revendication 1, **caractérisé en ce que**
- ledit corps forme deux chambres (46, 48) dudit vérin disposées de part et d'autre dudit piston (50) ; et
- ledit piston comprend une plaque (52) solidaire de l'extrémité proximale de ladite aiguille et un diaphragme (53) déformable de forme annulaire dont un bord (53b) est solidaire de la périphérie de ladite plaque (52) et dont l'autre bord (53a) est solidaire dudit corps (32).

3. Dispositif selon la revendication 2, **caractérisé en ce que** ledit corps comprend en outre une première conduite d'alimentation (58) de la première chambre (46) du vérin et une deuxième conduite d'alimentation (60) de la deuxième chambre (48) du vérin.

4. Dispositif selon la revendications 3, **caractérisé en ce qu'**il comporte des moyens (64, 66) pour relier alternativement chaque conduite à une source de gaz sous pression et à l'échappement et des moyens de commande (68) des moyens de mise en liaison.

5. Dispositif selon la revendication 4, **caractérisé en ce que** lesdits moyens de commande (68) commandent les moyens de liaison (64, 66) de telle manière que le cycle complet de déplacement de l'aiguille par rapport à la canule ait une durée constante et que, entre deux cycles consécutifs, l'extrémité distale (42) de l'aiguille (40) soit maintenue dans sa deuxième position.

6. Dispositif selon l'une quelconque des revendications 1 à 5, **caractérisé en ce que** l'extrémité distale (42) de ladite aiguille (40) est coupante.

7. Dispositif selon l'une quelconque des revendications 1 à 6, **caractérisé en ce que** l'extrémité proximale (40a) de ladite aiguille (40) est raccordée à des moyens d'aspiration.

## Claims

1. A vitrectomy device, comprising:
- a body (32);
- a canula (36) extending at one end of the body, said canula forming a distal end (36a) which is closed and provided with a window (38) in its lateral wall in proximity to the distal end;
- a needle (40) which is movable by an alternating movement within the canula, in order to periodically close said window;
- suction means which are connected to said needle; and
- a cylinder (44) provided with a movable piston (50) for controlling the movement of said needle;
said needle being moved by an alternating translational movement inside the canula;
- a tension spring (56) disposed between said body (32) and said piston (50) for bringing said needle (40) back into its second position;
said device being **characterised by** the facts that:
- said cylinder is double-acting;
- said piston of said cylinder is directly integrally combined with said needle and extends essentially perpendicularly to the needle's axis, the displacement of said piston being capable of moving said needle into a first position in which said needle closes said window (38) and into a second position in which the distal end (42) of said needle is removed from the distal end (36a) of said canula, so that said window remains open.

2. The device according to claim 1, **characterised in that**
- said body forms two chambers (46, 48) of said cylinder, one on either side of said piston (50); and
- said piston comprises a plate (52) which is integrally combined with the proximal end of said needle and a deformable annular diaphragm (53), one edge of which is integrally combined with the periphery of said plate (52) and the other edge (53a) of which is integrally combined with said body.

3. The device according to claim 2, **characterised in that** said body further comprises a first supply conduit (58) for the first chamber (46) and a second supply conduit (60) for the second chamber (48).

4. The device according to claim 3, **characterised in that** it comprises means (64, 66) for alternatingly connecting the conduits with a pressurised gas source and an exhaust and means (68) for controlling said connecting means.

5. The device according to claim 4, **characterised in that** said controlling means (68) control said connecting means (64, 66) in order to ensure the constant duration of a complete cycle of the needle's displacement in relation to said canula and to keep the distal end (42) of said needle (40) in its second position between two consecutive cycles.

6. The device according to any one of the claims 1 to 5, **characterised in that** the distal end (42) of said needle (40) is cutting.

7. The device according to any one of the claims I to 6, **characterised in that** the proximal end (40a) of said needle (40) is connected to said suction means.

## Patentansprüche

1. Vitrektomiegerät, das Folgendes umfasst:
- einen Körper (32);
- eine Kanüle (36), die sich an einem Ende des Körpers erstreckt, wobei die Kanüle ein distales Ende (36a) bildet, das verschlossen und mit einem Fenster (38) in seiner Seitenwand in der Nähe des distalen Endes versehen ist;
- eine Nadel (40), die durch eine alternierende Bewegung in der Kanüle bewegt wird, um das Fenster regelmäßig zu verschließen;
- Saugmittel, die mit der Nadel verbunden sind; und
- einen Zylinder (44), der mit einem bewegbaren Kolben (50) versehen ist, um die Bewegung der Nadel zu steuern;
wobei die Nadel durch eine alternierende Translationsbewegung im Inneren der Kanüle bewegt wird;
- eine Zugfeder (56), die zwischen dem Körper (32) und dem Kolben (50) angeordnet ist, und dazu dient, die Nadel (40) wieder in ihre zweite Position zu bringen;
wobei das Gerät, **dadurch gekennzeichnet ist, dass**:
- der Zylinder ein doppeltwirkender Zylinder ist;
- der Kolben des Zylinders direkt einstückig mit der Nadel ausgebildet ist und sich im Wesentlichen im rechten Winkel auf die Achse der Nadel erstreckt, wobei die Verschiebungen des Kolbens geeignet sind, um die Nadel in eine erste Position, in der die Nadel das Fenster (38) verschließt, und in eine zweite Position zu bringen, in der das distale Ende (42) der Nadel von dem distalen Ende (36a) der Kanüle entfernt ist, um das Fenster offen zu lassen.

2. Gerät nach Anspruch 1, **dadurch gekennzeichnet, dass**
- der Körper zwei Kammern (46, 48) des Zylinders bildet, die auf der einen und auf der anderen Seite des Kolbens (50) angeordnet sind; und
- der Kolben eine Platte (52), die einstückig mit dem proximalen Ende der Nadel ausgebildet ist, und eine verformbare, ringförmige Membran (53) umfasst, deren einer Rand (53b) einstückig mit dem Umfang der Platte (52) ausgebildet ist und deren anderer Rand (53a) einstückig mit dem Körper (32) ausgebildet ist.

3. Gerät nach Anspruch 2, **dadurch gekennzeichnet, dass** der Körper ferner einen ersten Kanal (58) zur Versorgung der ersten Kammer (46) des Zylinders und einen zweiten Kanal (60) zur Versorgung der zweiten Kammer (48) des Zylinders umfasst.

4. Gerät nach Anspruch 3, **dadurch gekennzeichnet, dass** es Mittel (64, 66) umfasst, um die Kanäle jeweils alternierend mit einer Druckgasquelle und einem Auslass zu verbinden, und Mittel (68) zur Steuerung der Mittel zur Verbindungsherstellung.

5. Gerät nach Anspruch 4, **dadurch gekennzeichnet, dass** die Steuerungsmittel (68) die Verbindungsmittel (64, 66) so steuern, dass der vollständige Zyklus der Verschiebung der Nadel in Bezug auf die Kanüle eine konstante Dauer aufweist und dass das distale Ende (42) der Nadel (40) zwischen zwei aufeinander folgenden Zyklen in der zweiten Position gehalten wird.

6. Gerät nach einem der Ansprüche 1 bis 5, **dadurch gekennzeichnet, dass** das distale Ende (42) der Nadel (40) eine Schneide aufweist.

7. Gerät nach einem der Ansprüche 1 bis 6, **dadurch gekennzeichnet, dass** das proximale Ende (40a) der Nadel (40) mit den Saugmitteln verbunden ist.
